# EUROPEAN PATENT SPECIFICATION

(11) **EP 2 934 524 B1**
(45) Date of publication and mention of the grant of the patent: **27.03.2019**
(21) Application number: 13864736.7
(22) Date of filing: 19.12.2013
(51) Int. Cl.: A61K 31/43, A61K 47/44, A61P 31/04

(54) **PENETHAMATE VETERINARY INJECTABLE FORMULATIONS**
PENETHAMATE INJIZIERBARE TIERÄRZTLICHE FORMULIERUNGEN
FORMULATIONS VÉTÉRINAIRES INJECTABLES DE PÉNÉTHAMATE

(30) Priority: 20.12.2012 NZ 60500512; 13.09.2013 NZ 61548513
(43) Date of publication of application: 28.10.2015
(73) Proprietor: Alleva Animal Health Limited, Auckland 0626 (NZ)
(72) Inventor: ALANY, Raid, Banstead Surrey SM7 1ET (GB); HOLMES, Robert William Lachlan, Auckland 0626 (NZ)
(74) Representative: Fry, David John
(86) International application number: PCT/NZ2013/000241
(87) International publication number: WO 2014/098624

(56) References cited:
- WO-A1-2014/014363
- GB-A- 2 084 572
- GB-A- 2 084 572
- US-A- 4 446 144
- US-A- 4 446 144
- US-A- 4 882 325
- AGROVETMARKET S.A.: 'Proxifen® 23 L.A. Injectable solution. Long Action Antibiotic - Non Steroidal Antiinflamatory Combination.' FORMULATION; PHARMACODYNAMIC - MECHANISM OF ACTION; DOSAGE AND ADMINISTRATION March 2008, pages 1 - 2, XP055263485 Retrieved from the Internet: <URL:http://www.agrovetmarket.com/Files/869 9b4a3-5b6a-40f4-a281-849192f802c5.pdf> [retrieved on 2014-04-10]

## Description

### Field

This invention relates to antibiotics and in particular to injectable formulations suitable for use in the treatment of lactating dairy cattle.

### Background

There are a wide variety of bacterial infections, which may infect domestic livestock. Of particular importance are those infections known to cause mastitis in lactating dairy cattle. To combat these infections farmers may treat with an antibiotic indicated for the particular infective organism.

Various antibiotics can be used for this purpose with beta-lactam antibiotics being particularly preferred.

Beta-lactam antibiotics include penicillin derivatives (penams), cephalosporins (cephems), monobactams, and carbapenems.

Penethamate hydriodide [CAS no. 808-71-9] (IUPAC Name: 2-(diethylamino)ethyl (2S,5R,6R)-3,3-dimethyl-7-oxo-6-[(2-phenylacetyl)amino]-4-thia-1-azabicyclo[3.2.0] heptane-2-carboxylate), is an ester of benzyl penicillin. Each gram is equivalent to 1 million international units (i.u.) of benzyl penicillin (penicillin G).

First recognised in 1948 and developed commercially in the 1950's (GB759603 patent - *"*Method for the production of esters of penicillin", 1956; Jensen, K. A., Dragsted, P. J., Kaier, E. J. and Fredericksen, E.: "Leocillin (Benzyl Penicillin-Diethylaminoethylester Hydriodide)," Acta Path. et microbiol.Scand., 28:407, 1951). Other documents such as US4446144, US4882325 and GB2084572 disclose a Penethamate hydroiodide veterinary injection formulation.

It is particularly preferred for use in the treatment of gram positive mastitis, respiratory infections, footrot and sub-acute mastitis.

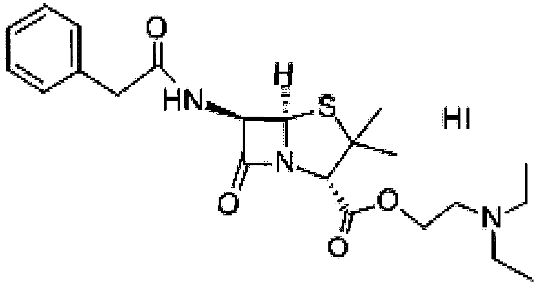

The advantageous properties of Penethamate hydriodide are that it is lipophilic salt of a weak base and upon injection, a high proportion will be present in the plasma and tissue in a non-ionised form. Because of this special property penethamate hydriodide has a special ability to diffuse through cell membranes, endothelia and tissue barriers, to the site of infection, where it is hydrolysed to the active benzyl penicillin. The levels of penicillin in the milk following injection of penethamate hydriodide are about 10 times higher than the levels following injection of other benzyl-penicillin preparations at an equivalent dose.

These properties mean this product is especially suitable for use in the systemic treatment of acute and sub-acute mastitis.

While Penethamate hydriodide is a drug of choice in combating bacterial infections in livestock it is not an easy product for the veterinarian or farmer to administer. Because it is not stable when mixed with water, it is provided in a vial containing 5 or 10 grams of penethamate hydriodide as an off white sterile powder for reconstitution.

Dosage of the product is typically 10-15mg/kg body weight daily, with the dose repeated for 1 to 5 days depending on the condition being treated. The dose is injected subcutaneously or intramuscularly. A 10mg dose of penethamate hydriodide powder would be administered by suspending in 30mL of water. This would result in a suspension containing 333mg/mL or 33.3% w/v.

The scope of the invention is defined by the claims. Any references in the description to methods of treatment refer to the compounds, pharmaceutical compositions and medicaments of the present invention for use in a treatment of the human (or animal) body by therapy (or diagnosis).

In New Zealand the meat withholding period for animals treated with penethamate hydriodide is 7 days, while milk from treated cattle must be discarded for not less than 48 hours after the last treatment of a 5g daily dose and 60 hours after the last treatment of a 10g treatment followed by two daily doses of a 5g treatment.

Commercial examples of Penethamate Hydriodide Injection include:
INGEL-MAMYZIN® powder + solvent for injection,
MAMYZIN® P powder + solvent for injection,
MAMYZIN® Parenteral powder + solvent for injection,
MAMYZIN® 5 powder + solvent for injection,
MAMYZIN® 10 powder + solvent for injection,
MAMYZIN® Vet. powder + solvent for injection,
PENETAVET® powder + solvent for injection
(all Boehringer Ingelheim, see www.mamvzin.com);
PENETHAJECT® (Bayer Bomac);
MASTIVET™ PARENTERAL (Divasa Farmavic);
YODIMASPEN™(Laboratorios Calier).

To use any of these products the farmer or veterinarian must follow this procedure:
1. Insert the needle of a syringe through the stopper of the water for injection vial
2. Use the syringe to withdraw the water
3. Pick up the vial containing the penethamate hydriodide powder
4. Insert the syringe needle through the stopper of the vial
5. Express the water from the syringe into the vial
6. Withdraw the syringe from the vial
7. Shake the vial vigorously to disperse the powder through the water
8. Reinsert the syringe needle through the stopper of the vial
9. Withdraw the required dose of the now mixed penethamate hydriodide suspension from the vial
10. Treat the animal

As will be appreciated this is a time consuming task. It is also a task that requires the user to be very adept at using the syringe to transfer the water for injection. The fact that it must be mixed before use, often in an on-farm environment, means that there are also increased opportunities to mix the powder incorrectly.

There is therefore a significant need for a simple ready to use formulation of penethamate hydriodide. By this term "ready to use" we mean that no other additives or reconstitution steps are needed - just that it can be shaken or agitated before being administered. Over the years, various attempts have been made to deliver an improved penethamate formulation; including formulations that are ready for injection rather than rely on reconstitution. Surprisingly, and despite the fact that penethamate has been available for almost 60 years none of these attempts have met with any degree of success.

### Object of the Invention

It is an object of the invention to provide veterinary injectable formulations that ameliorate some of the disadvantages and limitations of the known art or at least provide the public with a useful choice.

### Summary of the Invention

In a first aspect the invention provides an injectable veterinary suspension formulation comprising penethamate or a salt thereof and a substantially water free carrier comprising diethylene glycol monoethyl ether and/or solketal. Preferably this is a ready to use suspension formulation of penethamate consisting of penethamate hydriodide in a biocompatible oil or an organic solvent (or a combination thereof), and one or more pharmaceutically acceptable excipients suitable for intramuscular injection in domestic animals; in particular cattle.

Preferably the biocompatible oil is a vegetable oil.

The improvement over the prior art relates to the fact that the formulation is ready to use without additional preparation, aside from shaking to achieve adequate resuspension. Thus, the formulation can be supplied in a sealed container such as a vial allowing it to be easily shaken before use.

A pharmaceutical suspension is a coarse dispersion in which an internal phase is dispersed uniformly throughout an external phase. The internal phase consists of insoluble solid particles having a specific range of size which is maintained uniformly throughout the external phase with the aid of a single or combination of suspending agents.

The formulation of the penethamate ready to use suspension is based on use of a biocompatible oil or organic solvent as the external phase.

In a second aspect the invention provides a ready to use suspension formulation of penethamate or a salt thereof and a substantially water free carrier consisting predominately of a biocompatible oil or diethylene glycol monoethyl ether and/or solketal. (or a combination thereof).

Preferably the formulation is suitable for intramuscular injection in domestic animals; and in particular cattle.

Preferably the biocompatible oil is selected from the group comprising: canola oil, coconut oil, corn oil, cottonseed oil, olive oil, peanut oil, palm oil, sesame oil, soybean oil, safflower oil, sunflower oil, triglycerides (which are long chain fatty acid esters of (a) glycerol or (b) trihydroxy, dihydroxy, monohydroxy or even polyhydroxy compounds), and mixtures of triglycerides and fatty acids (such as MIGLYOL®, Cremer).

Preferably the organic solvent is selected from the group comprising: diethylene glycol monoethyl ether (abbreviated to DGMEE), Solketal (IUPAC name: (2,2-Dimethyl-1,3-dioxolan-4-yl)methanol).

Preferably the formulation contains a surfactant

Preferably, the surfactant is selected from sorbitan esters, polyoxyethylated sorbitan esters, polyethylene glycol stearate and lecithin
Preferably the injectable formulation also includes a moisture scavenger.

Preferably the formulation is provided in ready to use vials.

Optionally the formulation may include other actives. Preferably they are soluble in the carrier.

More preferably the other actives include analgesics.

Preferably the analgesic is selected from flunixin, tolfenamic acid, carprofen, meloxicam, metamizole and ketoprofen (IUPAC name: (*RS*)-2-(3-benzoylphenyl) propanoic acid). Preferably the analgesic is ketoprofen.

### Description of the Preferred Embodiment(s):

The following description will describe the invention in relation to preferred embodiments of the invention, namely veterinary injectable formulations.

The invention is in no way limited to these preferred embodiments as they are purely to exemplify the invention only and that possible variations and modifications would be readily apparent without departing from the scope of the invention.

The constituents listed in Table I are preferred in the invention:

| **Table I: Constituents preferred in Penethamate Hydriodide Suspension** | | |
|---|---|---|
| Constituent | Purpose | Preferred Concentration (w/v) |
| Penethamate hydriodide | Active ingredient | 10-70% |
| Oleaginous or Organic solvent vehicle/s | Carrier for the penethamate hydriodide particles | 20-90% |
| Miscible organic vehicle/s | Carrier for the penethamate hydriodide particles | 0-20% |
| Surfactant/s | Lower the surface tension of the liquid once injected | 0-20% |
| Buffer/s | Stabilize the suspension to a desired pH range | 0-5% |
| Suspending agent/s | Form film around particle and decrease interparticle attraction thereby imparting viscosity to the solution. | 0-10% |
| Wetting agent/s | Help disperse solids in the continuous liquid phase | 0-10% |
| Flocculating agent/s | Flocculate the Penethamate drug particles | 0-10% |
| Thickener/s | Increase the viscosity of suspension | 0-5% |
| Surface modifier/s | Adsorbed onto surface of active ingredient to help prevent particle agglomeration | 0-5% |
| Moisture scavenger/s | Absorb any residual water | 0-5% |
| Osmotic agents | Adjust osmotic pressure comparable to biological fluid | 0-5% |
| Local Anaesthetic/s | Reduce any pain associated with injection | 0-5% |
| Preservative/s | Prevent microbial growth | 0-5% |
| Antioxidant/s | Inhibit the oxidation of penethamate hydriodide in the formulation | 0-1% |

It is also preferred that water will essentially be absent from the formulation.

The invention demonstrates that a significantly more stable formulation is achieved when penethamate hydriodide is formulated as a liquid suspension in which water is essentially absent.

Because of the inherent water solubility of penethamate it has also proven possible to formulate a liquid suspension in which there is minimal difference between the pharmacokinetics of the existing marketed aqueous reconstitutable suspensions (such as MAMYZIN®, Boehringer Ingelheim; PENETHAJECT®, Bayer Bomac) and the ready to use suspension which is the subject of this invention.

Surprisingly the studies conducted in the development of the invention have also demonstrated that it is possible to include penethamate hydriodide in a ready to use suspension at a level at least as concentrated as the 333mg/ml concentration of the existing marketed aqueous reconstitutable suspension in its mixed condition.

Even more surprisingly it has been proven possible to formulate a suspension of penethamate hydriodide in which only organic solvents are used as the vehicle.

The composition preferably includes penethamate hydriodide micronized to a particle size of 0-5 microns. It can also include various alternative excipients. In particular those shown in Table II:

| **Table II: Example Constituents** | |
|---|---|
| Constituent | Examples |
| Active ingredient | Penethamate hydriodide |
| Oleaginous vehicle/s | A biocompatible oil of vegetable, animal or synthetic origin. Preferably selected from oils such as canola oil, coconut oil, corn oil, cottonseed oil, olive oil, peanut oil, palm oil, sesame oil, soybean oil, safflower oil, sunflower oil. Triglycerides, which are long chain fatty acid esters of glycerol, or mixtures of triglycerides and fatty acids (such as MIGLYOL®). Trihydroxy, dihydroxy, monohydroxy or even polyhydroxy compounds may be substituted for the glycerol. |
| Organic solvent vehicle/s | DGMEE, Solketal, Propylene Glycol |
| Oil miscible organic vehicle/s | Caprylic/Capric Triglycerides (e.g. CRODOMOL® GTCC, Croda), Isopropyl myristate, alcohol, glycerin, polyethylene glycol and polypropylene glycol |
| Surfactant/s | Lecithin, Sorbitan esters, polyoxyethylated sorbitan esters, polyethylene glycol stearate |
| Buffer/s | Such as citrate and phosphate buffering agents |
| Suspending agent/s | Such as gums (e.g., acacia, carrageenan, alginates and tragacanth), cellulosics (e.g., Methylcellulose, Hydroxyethylcellulose, Carboxymethylcellulose, Sodium Carboxymethylcellulose, Microcrystalline cellulose), and clays (e.g., bentonite and colloidal magnesium aluminum) |
| Wetting agent/s | Lecithin, Anionic (e.g., docusate sodium and sodium lauryl sulfate) and nonionic (polysorbates, polyoxamers, octoxynol-9) |
| Flocculating agent/s | Such as Sorbitan esters, polyoxyethylated sorbitan esters |
| Thickener/s | Such as gelatin, beeswax, hydrogenated oils, 12-hydroxystearin, aluminium stearate, lactose, natural gums and cellulose derivatives (such as those listed above as suspending agents) |
| Surface modifier/s | Such as gelatin, casein, lecithin, gum acacia, cholesterol, tragacanth, stearic acid, benzalkonium chloride, calcium stearate, glyceryl monostearate, cetostearyl alcohol, cetomacrogol emulsifying wax, sorbitan esters, polyoxyethylene alkyl ethers, polyoxyethylene castor oil derivatives, polyoxyethylene sorbitan fatty acid esters, polyethylene glycols, polyoxyethylene stearates, colloidal silicon dioxide, phosphates, sodium dodecylsulfate, carboxymethylcellulose calcium, carboxymethylcellulose sodium, methylcellulose, hydroxyethylcellulose, hydroxypropylcellu lose, hydroxypropylmethycellulose phthalate, noncrystalline cellulose, magnesium aluminum silicate, triethanolamine, polyvinyl alcohol, and polyvinylpyrrolidone |
| Moisture scavenger/s | Such as cross-linked polyvinylpyrolidone (CL-PVP or crospovidone USP/NF (e.g. POLYPLASDONE® XL and XL-10, Ashland), celluloses and cellulosic materials, such as purified cellulose, microcrystalline cellulose, methylcellulose, carboxymethylcellulose and sodium carboxymethylcellulose, food grade sources of α- and amorphous cellulose (e.g., REXCEL®), and powdered cellulose, silica, tribasic calcium phosphate, sodium carboxymethylcellulose (sodium CMC), croscarmellose sodium (e.g., AC-DI-SOL®,FMC), and the like. |
| Osmotic agents | Such as sodium chloride, sodium sulfate, dextrose, mannitol and glycerol |
| Local Anaesthetic/s | Lidocaine, Bupivacaine, Tetracaine, Procaine, Mepivacaine |
| Preservative/s | Such as methyl and propyl paraben, benzyl alcohol, chlorobutanol and thimerosal may be added |
| Antioxidant/s | Such as Ascorbic acid derivatives (e.g. ascorbic acid, erythorbic acid, sodium ascorbate), Thiol derivatives (e.g. thioglycerol, cysteine, acetylcysteine, cystine, dithioerythreitol, dithiothreitol, glutathione), Tocopherols, Butylated hydroxyanisole(BHA), Butylated hydroxytoluene (BHT), Sulfurous acid salts (e.g. sodium sulfate, sodium bisulfite, acetone sodium bisulfite, sodium metabisulfite, sodium sulfite, sodium formaldehyde sulfoxylate, and sodium thiosulfate), Nordihydroguaiaretic acid |

The wetting or dispersing agent may be present in an amount of about 0.01% (w/v) to about 1% (w/v) based on the total volume of the formulation. More typically, the wetting or dispersing agent may be present in an amount of about 0.01% (w/v) to about 0.5% (w/v), about 0.01% (w/v) to about 0.1% (w/v), or about 0.05% (w/v) to about 0.2% (w/v). Preferably, the wetting or dispersing agent may be present in an amount of about 0.05% w/v. Advantageously, the wetting or dispersing agent may comprise lecithin (such as PHOSPHOLIPON® 90H, Lipoid). In one embodiment, PHOSPHOLIPON® 90H may be present in an amount of about 0.01% to about 0.10% w/v, and even more advantageously, about 0.05% w/v.

Hydrophilic colloids such as Lecithin coat hydrophobic drug particles (in this case the penethamate hydriodide) in one or more than one layer. This provides hydrophillicity to the drug particles and facilitate wetting. This aids deflocculation of the suspension because force of attraction between the particles is reduced.

In a particularly preferred embodiment the PHOSPHOLIPON®, along with Polyoxyethylated sorbitan ester (TWEEN®, Croda), and Sodium Citrate are blended with the Penethamate hydriodide before incorporation into the liquid carrier solution. By such an inclusion at the API manufacturing site further processing at a formulation facility can be much simplified.

The constituents of a preferred oily suspension formulation are listed in Table III

| **Table III: Constituents of Preferred Formulation** | | |
|---|---|---|
| Constituent | Purpose | Concentration |
| Penethamate hydriodide | Active ingredient | 10-70% |
| Lecithin (e.g. PHOSPHOLIPON® E80) | Surfactant | <1% |
| Polyoxyethylated sorbitan ester (e.g. TWEEN® 80) () or Sorbitan ester (SPAN®, Croda) | Surfactant | 1% |
| Silicon dioxide (e.g. AEROSIL® 200) | Thixotropic agents | <1% |
| Sodium Citrate | Preservative | 0-5% |
| Citric acid | Buffer | 0-5% |
| Propylene glycol | Solvent | 0-30% |
| Methyl Paraben or benzyl alcohol | Preservative | 0-5% |
| BHT/BHA | Antioxidant | 0-1% |
| Caprylic/Capric Triglyceride (e.g. MIGLYOL® 812 and/or 840) | Carrier | 20-90% |

In this embodiment, the biocompatible oil vehicle comprises MIGLYOL® 812, a Caprylic/Capric Triglyceride. An example of an alternative carrier is CRODAMOL® GTCC, supplied by Croda Health Care.

A further and more simple embodiment would include penethamate, lecithin, Polyoxyethylated sorbitan ester (TWEEN®) and Citric Acid.

The constituents of a preferred organic solvent suspension formulation are listed in Table IV

| **Table IV: Constituents of Preferred Formulation** | | |
|---|---|---|
| Constituent | Purpose | Concentration |
| Penethamate hydriodide | Active ingredient | 10-70% |
| Lecithin (PHOSPHOLIPON® E80) | Surfactant | <1% |
| Polyoxyethylated sorbitan ester (TWEEN® 80) or Sorbitan ester (SPAN®) | Surfactant | 1% |
| Silicon dioxide (AEROSIL® 200) | Thixotropic agents | <1% |
| Sodium Citrate | Preservative | 0-5% |
| Citric acid | Buffer | 0-5% |
| Methyl Paraben or benzyl alcohol | Preservative | 0-5% |
| BHT/BHA | Antioxidant | 0-1% |
| Solketal and/or DGMEE | Carrier | 20-90% |

### Method of Manufacture:

The suspension of the present invention may be prepared by any method known in the art for the preparation of injectable suspensions. All such methods involve the active ingredient being present in a suitable solid form and suspension thereof in a liquid vehicle. However, if the formulation contains lecithin (such as PHOSPHOLIPON®), the lecithin may be added to the penethamate hydriodide as a dry powder or via a heating and cooling step.

A manufacturing facility suitable for producing sterile products must be used if one is making this composition as an injectable for commercial use. Also, all manufacturing equipment and packaging components should be sterilized when making the suspension for administration by injection.

In the present invention, it is desirable to have as little water in the formulation as possible. The presence of water may cause a reduction in the shelf life of the formulation.

A number of alternative processing methods can be used to prepare the formulation. For example:

### Method 1

1. Blend penethamate hydriodide, Lecithin (PHOSPHOLIPON®), Sodium citrate acid, Polyoxyethylated sorbitan ester (TWEEN®) or Sorbitan ester (SPAN®) in the sterile dry powder
2. Place a volume of the carrier oil or solvent (namely diethylene glycol monoethyl ether and/or solketal) into a vessel
3. Add the penethamate hydriodide blend
4. Mix well and homogenize
5. Fill into sterile bottles.
6. Cap seal and label.
7. Optionally sterilise by gamma irradiation

### Method 2:

1. Mix all excipients in the carrier oil or solvent, (namely diethylene glycol monoethyl ether and/or solketal), use heat if needed.
2. Sterile filter into sterile vessel.
3. Allow to cool
4. Add sterile Penethamate Hydriodide, mix homogenize and fill into sterile bottles. Cap seal and label.
5. Optionally sterilize by gamma irradiation.

### Method 3: (not in the scope of the claims)

1. Place a volume of the oil into a vessel
2. Heat above 100°C
3. Add the lecithin (PHOSPHOLIPON®) and stirred until dissolved.
4. Allow to cool
5. Add and mix the Polyoxyethylated sorbitan ester (TWEEN®) or Sorbitan ester (SPAN®)
6. Add the penethamate hydriodide
7. Mix well and homogenize
8. Fill into sterile bottles.
9. Cap seal and label.
10. Optionally sterilize by gamma irradiation

### Formulation development

### Formulation development for penethamate suspensions

An attempt was made to formulate penethamate hydriodide as a suspension in oily solvents. Trial batches were made using the following oils: medium chained triglyceride (caprylic/capric triglycerides, CRODAMOL® GTCC), MIGLYOL® 840 (propylene glycol dicaprylate / dicaprate), glycerol triacetate, ethyl oleate and benzyl benzoate. Methyl paraben was added as a preservative. Surfactant(s) such as egg lecithin, polyoxyethylated sorbitan ester (TWEEN® 80) and/or sorbitan ester (SPAN® 80) were/was added as dispersant(s). Benzyl alcohol or propylene glycol was included in some of the batches as a co-solvent. About 16.7 g of penethamate was added to 50 ml oily vehicle, and homogenized at 5000 rpm for 10 min.

All formulations disclosed in Table V are not is the scope of the claims.

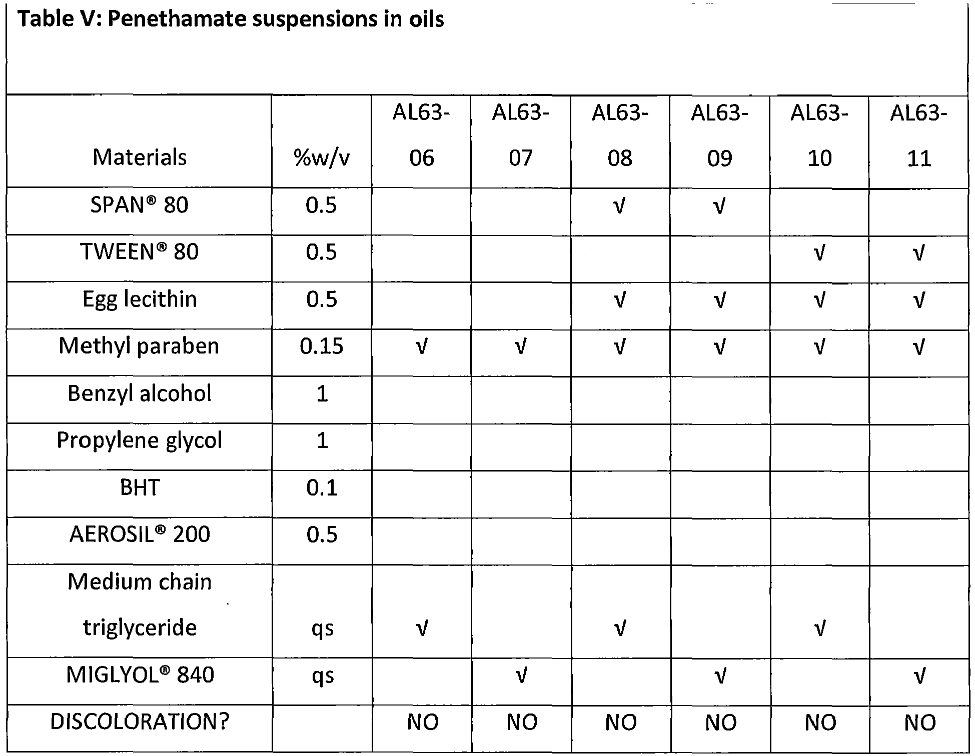

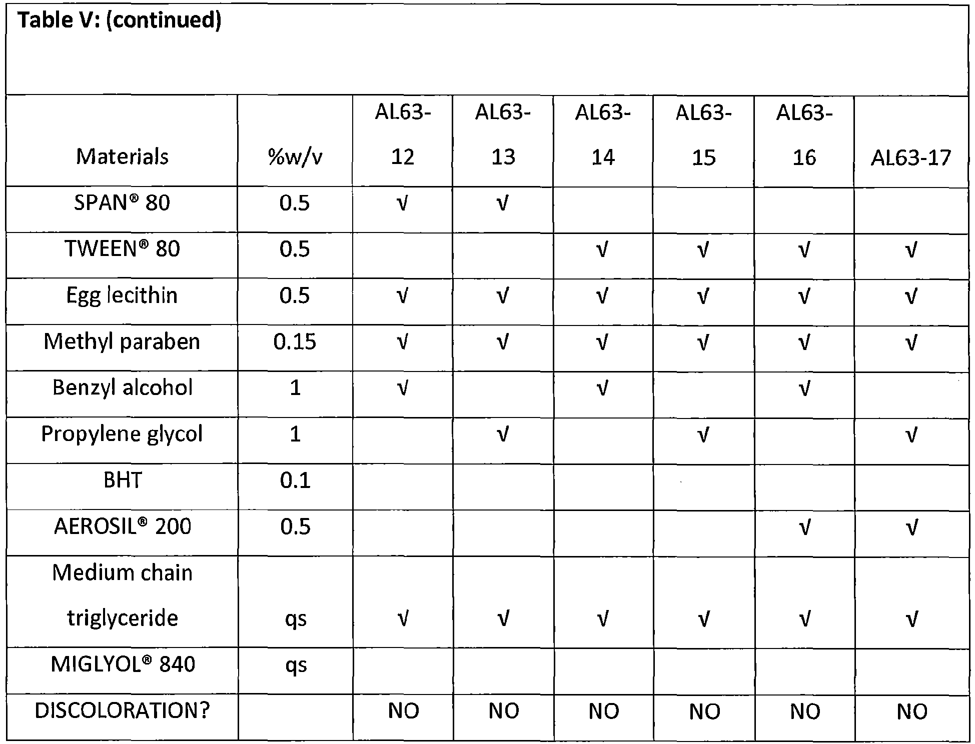

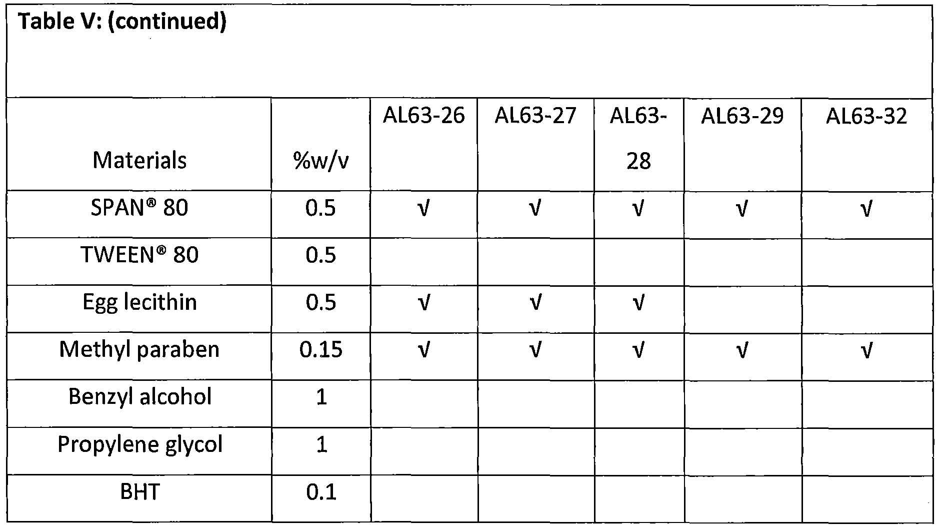

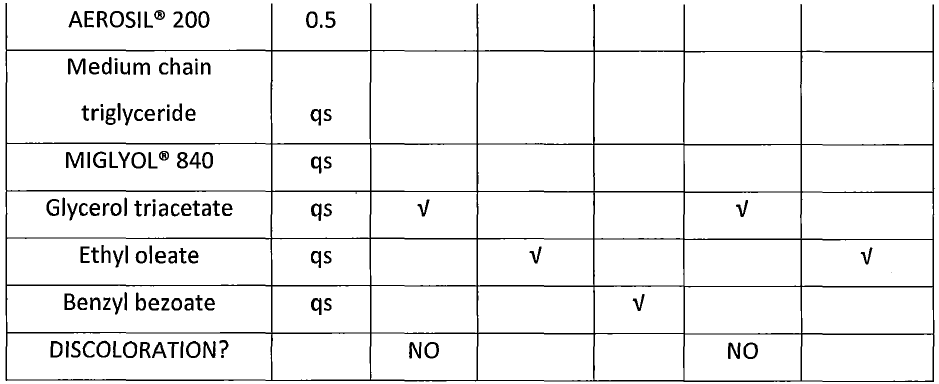

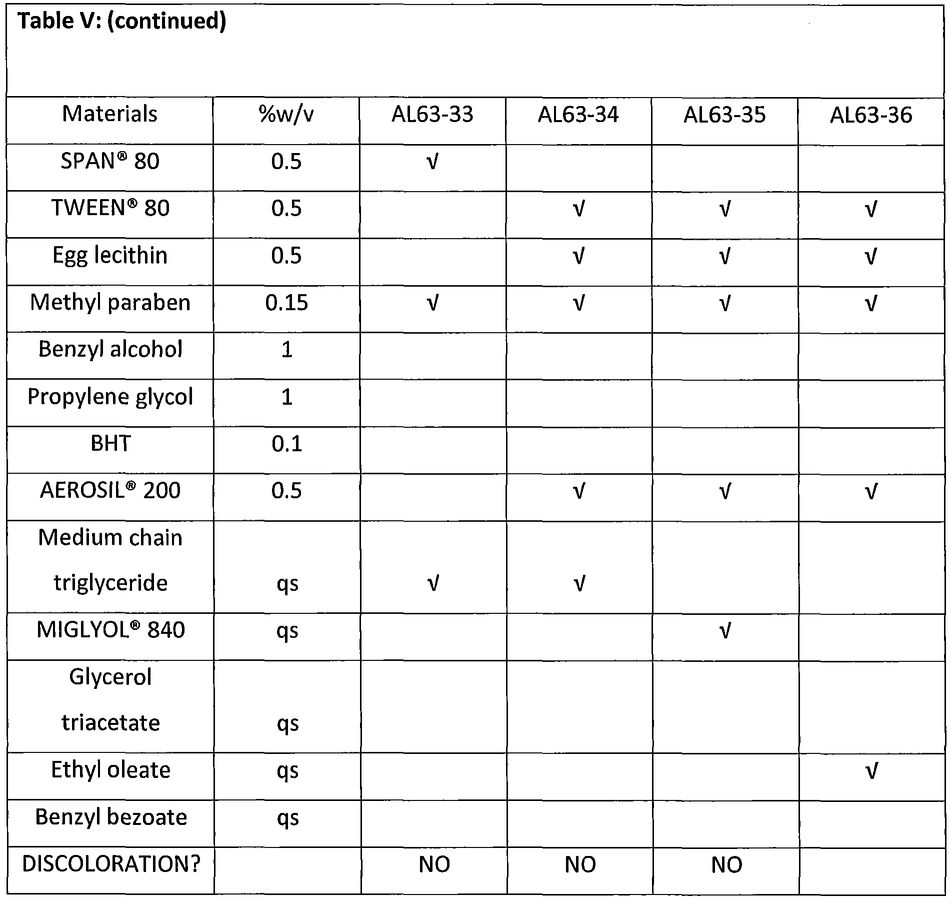

It was noticed that medium chain triglyceride, Propylene glycol dicaprylate/dicaprate (MIGLYOL® 840) and glycerol triacetate may be more suitable oils for penethamate oily suspensions, as the corresponding batches showed no significant discolouration when prepared.

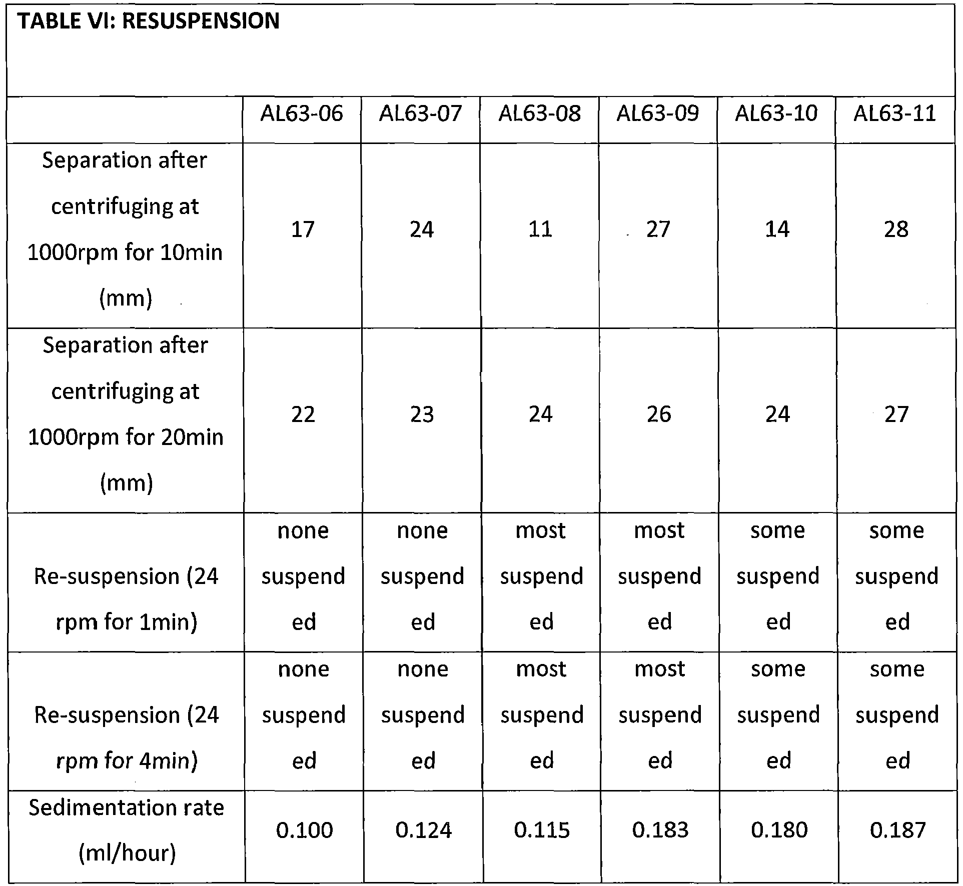

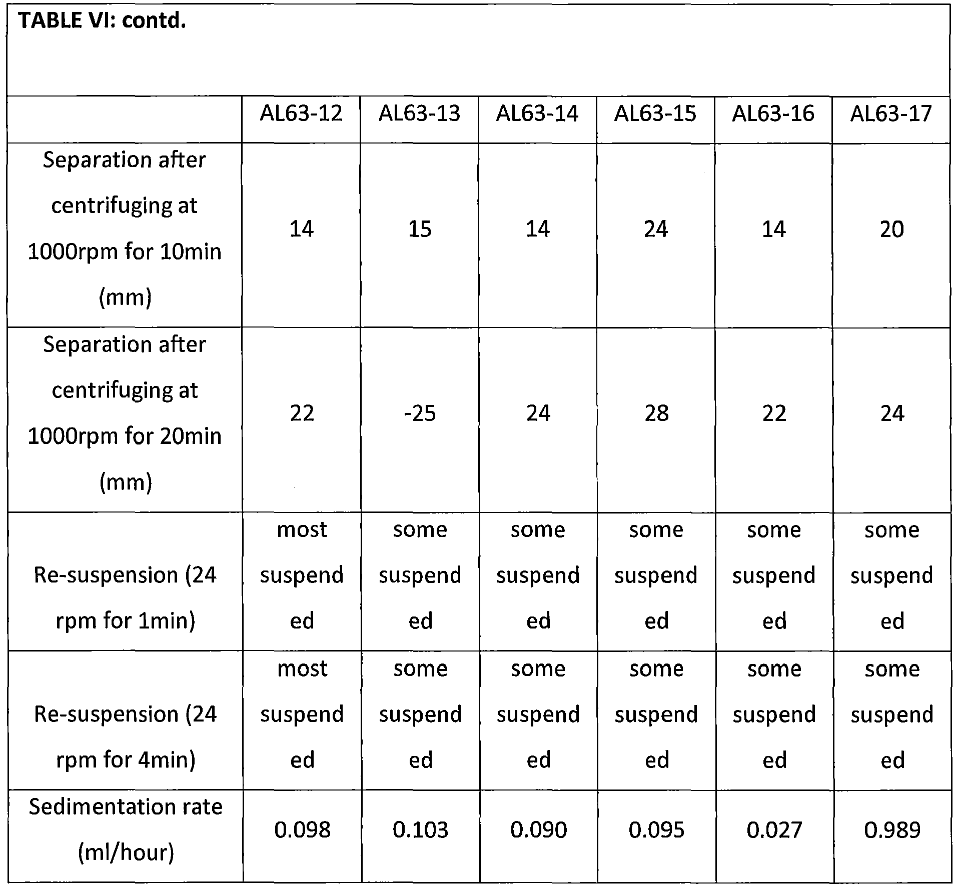

All batches were observed for discolouration. Only a few batches showed no sign of discolouration after one month at room temperature, these were AL63-06, AL63-07, AL63-10, AL63-11, AL63-29 and AL63-33. This demonstrates that the combination of SPAN® 80/egg lecithin, TWEEN® 80/ egg lecithin, TWEEN® 80/egg lecithin/benzyl alcohol, and TWEEN® 80/egg lecithin/propylene glycol may interact with formulation ingredients and result in discolouration.

The batches without discolouration were subjected to manual re-suspension studies. The batch AL63-08 was easier to resuspend than the other tested batches.

This can be seen from the attached two Figures which are photographs of actual batches before and after inversion.

Further, butylated hydroxytoluene (BHT) was introduced into the formulations as an antioxidant, as shown in Table VII. None of the batches containing BHT showed discolouration, indicating that BHT may be efficient in avoiding discolouration.

All formulations disclosed in Table VII are not is the scope of the claims

| **Table VII: Penethamate suspensions in oils containing BHT** | | | | | | |
|---|---|---|---|---|---|---|
| Materials | %w/v | AL63-37 | AL63-47 | AL63-48 | AL63-49 | AL63-50 |
| SPAN® 80 | 0.5 | √ | √ | | √ | |
| TWEEN® 80 | 0.5 | | | √ | | √ |
| Egg lecithin | 0.5 | √ | | | | |
| Methyl paraben | 0.15 | √ | √ | √ | √ | √ |
| BHT | 0.1 | √ | √ | √ | √ | √ |
| AEROSIL® 200 | 0.5 | | | | | |
| Medium chain triglyceride | qs | √ | | | | |
| MIGLYOL® 840 | qs | | √ | √ | | |
| Glycerol triacetate | qs | | | | √ | √ |

Stability studies in which the formulations were subjected to elevated temperatures demonstrated that the oily suspensions were highly stable.

### Formulation development for penethamate suspension in organic solvents

Following indications of success with oily vehicles it was speculated that it may be possible to use organic solvents as potential vehicles for a penethamate suspension. Solubility of penethamate was estimated at the concentrations of 0.2% w/w and 1.0% w/w, in the following solvents: glycerine, Solketal, polyethylene glycol 200 (PEG 200), diethylene glycol butyl ether (DGBE), propylene glycol (PG), glycofural, diethylene glycol monomethyl ether (DGME), diethylene glycol methyl ethyl ether (DGMEE), dimethyl isosorbide (DMI), 2-pyrrolidone (2-pyrol), N-methyl-2-pyrrolidone (NMP), dimethyl acetamide (DMA), glycerol formal (GF), benzyl alcohol (BA), ethyl lactate and dimethyl sulfoxide (DMSO). The resulting solutions/dispersions were observed for discolouration, which is an indication of instability in such solvents.

The solvents in which 0.2% w/w and 1.0% w/w penethamate showed no discolouration and minimal solubility were selected as the solvent candidates for the formulation development for penethamate suspensions in organic vehicles. The selected solvents were glycerine, Solketal, propylene glycol, glycerol formal and DGMEE.

The following formulations (as shown in Table VIII) were selected for the development of penethamate suspensions in organic vehicles, because they showed minimum physical changes in earlier work. Each of them was divided into two portions and stored at 2-8°C and 55°C for 2 weeks, and then analysed for penethamate content (as shown in Table IX). The results demonstrate that propylene glycol, Solketal and DGMEE were promising vehicles for penethamate suspensions, compared to the other tested organic vehicles.

| **Table VIII: Penethamate suspensions in organic vehicles** | | | | | | | |
|---|---|---|---|---|---|---|---|
| Materials | AL63-43 | AL63-44 | AL63-45 | AL63-46 | AL63-51 | AL63-52 | AL63-53 |
| BHT | - | - | - | 0.01g | - | - | - |
| GF | - | - | - | - | - | - | - |
| Glycerine | - | 10g | 5g | 5g | - | - | - |
| PG | 10g | - | 5g | 5g | - | - | 10g |
| Solketal | - | - | - | - | 10g | - | - |
| DGMEE | - | - | - | - | - | 10g | - |
| Penethamate | 3.3g | 3.3g | 3.3g | 3.3g | 3.3g | 3.3g | 3.3g |

| **Table IX: Penethamate assay in penethamate suspensions in organic vehicles** | | | | |
|---|---|---|---|---|
| BN | Solvent | % Penethamate | %BHT | Recovery (%) |
| AL63-43 | PG | 3.3g in 10ml | - | 61.65 |
| AL63-44 | Glycerin | 3.3g in 10ml | - | 81.86 |
| AL63-45 | PG/Glycerin | 3.3g in 10ml | - | 62.02 |
| AL63-46 | PG/Glycerin | 3.3g in 10ml | 0.1 | 33.45 |
| AL63-51 | Solketal | 3.3g in 10ml | - | 99.81 |
| AL63-52 | DGMEE | 3.3g in 10ml | - | 96.84 |
| AL63-53 | PG | 3.3g in 10ml | - | 66.03 |

To confirm that Solketal and DGMEE were suitable solvents for penethamate suspensions, the following batches were prepared and subjected to further stress studies (as shown in Table X). Corresponding batches containing BHT were also made and tested for comparison. The results (as shown in Table XI) were very comparable to those shown in table IX. Solketal and DGMEE are the most suitable organic vehicles for penethamate suspensions, with about 100% and 97% recovery of penethamate respectively. Moreover, the addition of BHT did not seem to enhance the stability of penethamate suspensions in the organic vehicles as they did in the oil-based suspensions.

| **Table X: Penethamate suspensions in propylene glycol, Solketal and DGMEE** | | | | | | |
|---|---|---|---|---|---|---|
| Materials | AL63-54 | AL63-55 | AL63-56 | AL63-57 | AL63-58 | AL63-59 |
| BHT | - | - | - | 0.1 | 0.1 | 0.1 |
| Solketal | 10g | - | - | 10g | - | - |
| DGMEE | - | 10g | - | - | 10g | - |
| PG | - | - | 10g | - | - | 10g |
| Penethamate | 3.3g | 3.3g | 3.3g | 3.3g | 3.3g | 3.3g |

| **Table XI: Penethamate assay in penethamate suspensions in propylene glycol, Solketal and DGMEE** | | | | |
|---|---|---|---|---|
| BN | Solvent | % Penethamate | %BHT | Recovery (%) |
| AL63-54 | Solketal | 3.3g in 10ml | - | 100.79 |
| AL63-55 | DGMEE | 3.3g in 10ml | - | 96.83 |
| AL63-56 | PG | 3.3g in 10ml | - | 65.33 |
| AL63-57 | Solketal | 3.3g in 10ml | 0.1 | 100.44 |
| AL63-58 | DGMEE | 3.3g in 10ml | 0.1 | 95.92 |
| AL63-59 | PG | 3.3g in 10ml | 0.1 | 61.70 |

Further, penethamate suspensions in the combination of Solketal and DGMEE were formulated (as shown in Table XII) and subjected to stress studies. The results are shown in Table XIII.

| **Table XII: Penethamate suspensions in the combination of Solketal and DGMEE** | | |
|---|---|---|
| Materials | AL63-60 | AL63-61 |
| BHT | - | 0.1 |
| Solketal | 5g | 5g |
| DGMEE | 5g | 5g |
| Penethamate | 3.3g | 3.3g |

| **Table XIII: Penethamate assay in organic vehicles suspensions in the combination of Solketal and DGMEE** | | | | |
|---|---|---|---|---|
| BN | Solvent | % Penethamate | %BHT | Recovery (%) |
| AL63-60 | Solketal/DGMEE | 3.3g in 10ml | - | 96.58 |
| AL63-61 | Solketal/DGMEE | 3.3g in 10ml | 0.1 | 96.53 |

The suspensions produced by the above methods demonstrate all the characteristics required for a suitable ready to use penethamate hydriodide formulation:
- Easy to resuspend without the need for vigorous shaking
- Good flowability and syringeability
- Uniform appearance
- Physical, Chemical and Microbiological stability
- Easy to process and sterilise

Injection site tolerance studies were undertaken in which animals were treated with the various embodiments of the invention. In all cases the formulations were well tolerated with no adverse reactions.

### Suspension formulations of Penethamate with other actives.

Surprisingly and quite unexpectedly it has also proven possible to incorporate an analgesic agent in the formulation including the organic solvents. This has been achieved by successfully dissolving the agent in the solvent blend while the penethamate remains suspended. It is anticipated that other analgesic agents and other soluble actives may also be used. To prepare the formulation the analgesic agent is firstly dissolved in the solvent blend. Once this is achieved the penethamate is added.

Examples of such formulations are as follows:

| **Penethamate/Ketoprofen Formulation** | |
|---|---|
| **Materials** | **Concentration** |
| Penethamate | 33% |
| Ketoprofen | 10% |
| BHT | 0.1% |
| DGMEE | 25% |
| Solketal | To vol |

| **Penethamate/Ketoprofen Formulation** | |
|---|---|
| **Materials** | **Concentration** |
| Penethamate | 33% |
| Ketoprofen | 10% |
| BHT | 0.1% |
| Solketal | To vol |

Such a product has significant advantages in that it enables co-administration of a bacterial agent and analgesic to animals that would benefit from such a treatment. Given that the bacterial agent and analgesic agent typically require quite different formulation conditions the invention of a system able to incorporate both agents is a useful milestone. It is anticipated that other analgesic agents such as flunixin, tolfenamic acid, carprofen, meloxicam and metamizole might also be used.

### Advantages:

The invention provides a ready to use suspension formulation of penethamate.

The ready to use suspension formulation is suitable for intramuscular injection in domestic animals; in particular cattle.

It needs only shaking of the vial (or other container) to achieve adequate re-suspension and is thus easier and quicker to use on the farm then previous injectable forms of penethamate.

### Definitions:

It is acknowledged that the term 'comprise' may, under varying jurisdictions, be attributed with either an exclusive or an inclusive meaning. For the purpose of this specification, and unless otherwise noted, the term 'comprise' shall have an inclusive meaning - i.e. that it will be taken to mean an inclusion of not only the listed components it directly references, but also other non-specified components or elements. This rationale will also be used when the term 'comprised' or 'comprising' is used in relation to one or more steps in a method or process.

The phrase "substantially water free" used in connection with the formulations of this invention is to be understand that water is not an intentional ingredient of the formulation and that whilst water should not be present in the formulation that if some water is detected in the formulation that an amount of less than 1% of the formulation will be within the meaning of this phrase.

MIGLYOL® is a trademark for various distillation fractions of coconut oil, typically comprising a mixture of triglycerides of C₈ and triglycerides of C₁₀ fatty acids.

"Ready to use" in relation to the formulation means that no other additives or reconstitution steps are needed - just that the formulation can be shaken or agitated before being administered.

### Variations:

It will of course be realised that while the foregoing has been given by way of illustrative example of this invention, all such and other modifications and variations thereto as would be apparent to persons skilled in the art are deemed to fall within the broad scope and ambit of this invention as is hereinbefore described.

## Claims

1. An injectable veterinary suspension formulation comprising penethamate or a salt thereof and a substantially water free carrier comprising diethylene glycol monoethyl ether and/or solketal.

2. An injectable veterinary suspension formulation as claimed in claim 1 wherein the penethamate is present as penethamate hydriodide, and the formulation includes one or more pharmaceutically acceptable excipients and is for intramuscular injection in domestic animals; in particular cattle.

3. An injectable veterinary suspension formulation as claimed in claim 1 or 2 wherein the carrier includes a biocompatible oil selected from the group: canola oil, coconut oil, corn oil, cottonseed oil, olive oil, peanut oil, palm oil, sesame oil, soybean oil, safflower oil, sunflower oil, triglycerides and mixtures of triglycerides and fatty acids.

4. An injectable veterinary suspension formulation as claimed in claim 3, wherein the mixture of triglyceride and fatty acids is selected from:
• propylene glycol dicaprylate / dicaprate; and
• caprylic/capric triglyceride.

5. An injectable veterinary suspension formulation as claimed in claim 3, wherein the triglycerides are selected from long chain fatty acid esters of (a) glycerol or (b) trihydroxy, dihydroxy, monohydroxy or polyhydroxy compounds.

6. An injectable veterinary suspension formulation as claimed in any preceding claim which also includes a moisture scavenger.

7. An injectable veterinary suspension formulation as claimed in claim 6, wherein the moisture scavenger is selected from cross-linked polyvinylpyrolidone, celluloses and cellulosic materials, food grade sources of α- and amorphous cellulose, powdered cellulose, silica, tribasic calcium phosphate, sodium carboxymethylcellulose, and croscarmellose sodium.

8. An injectable veterinary suspension formulation as claimed in claim 7, wherein the moisture scavenger is selected from purified cellulose, microcrystalline cellulose, methylcellulose, carboxymethylcellulose and sodium carboxymethylcellulose.

9. An injectable veterinary suspension formulation as claimed in any preceding claim which also includes an analgesic.

10. An injectable veterinary suspension formulation as claimed in claim 9 wherein the analgesic is ketoprofen.

## Patentansprüche

1. Injizierbare Veterinärsuspensionsformulierung, umfassend Penethamat oder ein Salz davon und einen im Wesentlichen wasserfreien Träger, umfassend Diethylenglycolmonoethylether und/oder Solketal.

2. Injizierbare Veterinärsuspensionsformulierung nach Anspruch 1, wobei das Penethamat als Phenethamathydrojodid vorhanden ist und die Formulierung einen oder mehrere pharmazeutisch annehmbare Hilfsstoffe beinhaltet und der intramuskulären Injektion in Heimtiere, insbesondere Rind, dient.

3. Injizierbare Veterinärsuspensionsformulierung nach Anspruch 1 oder 2, wobei der Träger ein biokompatibles Öl beinhaltet, das aus der folgenden Gruppe ausgewählt ist: Rapsöl, Kokosöl, Maisöl, Baumwollsamenöl, Olivenöl, Erdnussöl, Palmöl, Sesamöl, Sojaöl, Safloröl, Sonnenblumenöl, Triglyceriden und Mischungen aus Triglyceriden und Fettsäuren.

4. Injizierbare Veterinärsuspensionsformulierung nach Anspruch 3, wobei die Mischung aus Triglycerid und Fettsäuren aus Folgendem ausgewählt ist:
• Propylenglycoldicaprylat/-dicaprat; und
• Capryl-/Capric-Triglycerid.

5. Injizierbare Veterinärsuspensionsformulierung nach Anspruch 3, wobei die Triglyceride aus langkettigen Fettsäureestern von (a) Glycerol oder (b) Trihydroxy-, Dihydroxy-, Monohydroxy- oder Polyhydroxy-Verbindungen ausgewählt sind.

6. Injizierbare Veterinärsuspensionsformulierung nach einem vorhergehenden Anspruch, die auch einen Feuchtigkeitsfänger beinhaltet.

7. Injizierbare Veterinärsuspensionsformulierung nach Anspruch 6, wobei der Feuchtigkeitsfänger aus vernetztem Polyvinylpyrolidon, Cellulosen und cellulosischen Materialien, lebensmittelgeeigneten Quellen von α- und amorpher Cellulose, Pulvercellulose, Siliziumdioxid, tribasischem Calciumphosphat, Natriumcarboxymethylcellulose und Croscarmellose-Natrium ausgewählt ist.

8. Injizierbare Veterinärsuspensionsformulierung nach Anspruch 7, wobei der Feuchtigkeitsfänger aus gereinigter Cellulose, mikrokristalliner Cellulose, Methylcellulose, Carboxymethylcellulose und Natriumcarboxymethylcellulose ausgewählt ist.

9. Injizierbare Veterinärsuspensionsformulierung nach einem vorhergehenden Anspruch, die auch ein Analgetikum beinhaltet.

10. Injizierbare Veterinärsuspensionsformulierung nach Anspruch 9, wobei das Analgetikum Ketoprofen ist.

## Revendications

1. Une préparation de suspension vétérinaire injectable comprenant du pénéthamate ou un sel de celui-ci et un support substantiellement exempt d'eau comprenant un éther monoéthylique de diéthylène glycol et/ou du solkétal.

2. Préparation de suspension vétérinaire injectable selon la revendication 1 dans laquelle le pénéthamate est présent sous forme d'iodhydrate de pénéthamate, et la préparation inclut un ou plusieurs excipients pharmaceutiquement acceptables et est destinée à une injection intramusculaire dans des animaux domestiques ; en particulier du bétail.

3. Préparation de suspension vétérinaire injectable selon la revendication 1 ou 2 dans laquelle le support inclut une huile biocompatible sélectionnée parmi le groupe : huile de colza, huile de noix de coco, huile de maïs, huile de coton, huile d'olive, huile d'arachide, huile de palme, huile de sésame, huile de soja, huile de carthame, huile de tournesol, triglycérides et mélanges de triglycérides et d'acides gras.

4. Préparation de suspension vétérinaire injectable selon la revendication 3, dans laquelle le mélange de triglycéride et d'acides gras est sélectionné parmi :
• dicaprylate / dicaprate de propylène glycol ; et
• triglycéride caprylique/caprique.

5. Préparation de suspension vétérinaire injectable selon la revendication 3, dans laquelle les triglycérides sont sélectionnés parmi des esters d'acides gras à chaîne longue de (a) glycérol ou de (b) composés trihydroxy, dihydroxy, monohydroxy ou polyhydroxy.

6. Préparation de suspension vétérinaire injectable selon l'une quelconque des revendications précédentes qui inclut aussi un capteur d'humidité.

7. Préparation de suspension vétérinaire injectable selon la revendication 6, dans laquelle le capteur d'humidité est sélectionné parmi la polyvinylpyrolidone réticulée, des celluloses et des matières cellulosiques, des sources de catégorie alimentaire de α-cellulose et de cellulose amorphe, la cellulose en poudre, la silice, le phosphate de calcium tribasique, la carboxyméthylcellulose sodique, et la croscarmellose sodique.

8. Préparation de suspension vétérinaire injectable selon la revendication 7, dans laquelle le capteur d'humidité est sélectionné parmi la cellulose purifiée, la cellulose microcristalline, la méthylcellulose, la carboxyméthylcellulose et la carboxyméthylcellulose sodique.

9. Préparation de suspension vétérinaire injectable selon l'une quelconque des revendications précédentes qui inclut aussi un analgésique.

10. Préparation de suspension vétérinaire injectable selon la revendication 9 dans laquelle l'analgésique est le kétoprofène.
